# EUROPEAN PATENT SPECIFICATION

(11) **EP 0 429 608 B1**
(45) Date of publication and mention of the grant of the patent: **15.03.1995**
(21) Application number: 90908963.3
(22) Date of filing: 08.06.1990
(51) Int. Cl.: C08F 220/06, C08F 8/14, C08F 2/32, A61L 15/00

(54) **PROCESSES FOR PRODUCING HIGHLY WATER ABSORPTIVE RESINS**
VERFAHREN ZUR HERSTELLUNG VON HARZEN MIT HOHER WASSERABSORPTION
PROCEDES POUR LA PRODUCTION DE RESINES A ABSORPTION D'EAU ELEVEE

(30) Priority: 21.06.1989 KR 898596; 21.06.1989 KR 898597; 03.07.1989 KR 899405; 03.07.1989 KR 899406; 27.07.1989 KR 8910672; 27.07.1989 KR 8910673; 07.10.1989 KR 8914440
(43) Date of publication of application: 05.06.1991
(73) Proprietor: LUCKY, LTD., Seoul 150-010 (KR)
(72) Inventor: CHOI, Su Beom, Daejeon-si (KR); LEE, Hyung Mann, Daejeon-si (KR); KIM, Myung, Joong, Yoosung-gu Daejeon-si (KR); JANG, Tae Hwan, Daejeon-si (KR)
(74) Representative: Smulders, Theodorus A.H.J., Ir.
(86) International application number: PCT/KR90/00005
(87) International publication number: WO 90/15829

(56) References cited:
- DE-A- 3 617 311
- DE-A- 3 628 482
- US-A- 4 340 706
- US-A- 4 497 930

## Description

### BACKGROUND OF THE INVENTION

### Field of the Invention

The present invention relates to a process for producing highly water absorptive resins having excellent water absorbency and saline solution absorbency. More particularly, it relates to processes for producing highly water absorptive resins having a high water absorption rate and water absorptive capacity, and an excellent gel-strength.

### Description of the Prior Art

Various water absorbing materials such as sponge, pulp, paper and the like, are well known in the art. Also various synthetic products made by graft polymerization of materials containing a hydrophilic group, such as -OH, -NH₂, -COOH, and the like, have been used conventionally. However, such water absorbing materials, e.g. a sponge, a pulp, a paper are characterized with physical mechanism for absorbing water so that such materials have defects in that the majority of the absorbed water can he easily squeezed out by the application of external pressure. In recent years, in order to solve these defects, synthetic water-absorptive products having particular physical and chemical mechanisms have been developed. The majority of such synthetic products are crosslinked polyacrylic acid salt, or polymethacrylic acid salt, crosslinked polyacrylic acid-methacrylic acid copolymer salt, crosslinked saponification products of starch-acrylonitrile graft copolymer, and cellulose and acrylate-grafted copolymer. Such grafted products are on the market as sanitary napkins, sanitary pads, diapers in the sanitary field, water absorbing containers in the civil and gardening field, and anti-dewdrop agents in the construction field.

However, although saponificated products of starch-acrylonitrile graft copolymer has a relatively high deionized water-absorbency, the saline-solution thereof is poor. Also, it is impossible to be stored for a long time because its main component is starch.

On the other hand, partially crosslinked polyacrylic acid salt has a high water absorption rate and capacity in saline solution as well as in deionized water, and it can be stored for a long period of time. Particulary, it is readily available commercially, therefore, alkali metal acrylate is used as a starting material to prepare a water absorptive resin in the present invention.

As the process for polymerizing acrylic acid and alkali acrylate, there have been known various processes such as bulk polymerization, aqueous solution polymerization, spray polymerization, inverse emulsion polymerization, inverse suspension polymerization, and the like. But, in case of using the processes except for the inverse emulsion polymerization and the inverse suspension polymerization, it is difficult to remove the heat of polymerization, and the viscosity of polymerization mixture becomes too high to carry out the production of general polymers. Moreover, according to these processes it is difficult to obtain particulate polymer.

An example of the inverse emulsion polymerization process is disclosed in US patent NO. 3,284,393. When, for example, acrylic acid is used as the starting material, the obtained polymer is insoluble in water and does not have such an absorbency that the polymer can be called a water absorptive resin even if it is neutralized with an alkali such as sodium hydroxide or the like.

As a process for producing an acrylic acid-alkali metal acrylate polymer having water absorbency, the inverse suspension polymerization process is mentioned in Japanese Patent Publication No. 79-30710. According to this process, a water absorptive polymer is prepared through a stable reaction by the use of a sorbitan fatty acid ester having a HLB(Hydrophilic-Lipophilic Balance) value of 3∼6. The resin thus obtained has a high deionized water absorbency corresponding to 400∼500 times its own weight, however, the saline solution-absorbency of said water absorptive resin is as low as 35∼50 times its own weight.

In US patent No. 4,497,930, a method is disclosed for producing a water absorbent polymer comprising the steps of subjecting acrylic acid and sodium acrylate to polymerization in the presence of a sorbitan fatty acid ester having a HLB value of 3∼6 as a dispersing agent, and adding a crosslinking agent with stirring in an organic solvent to crosslink the surface of the resulting polymer. However, it is a disadvantage that the water absorptive resin prepared by this method is unsatisfactory in water absorptive capacity, because the polymerization reaction is carried out at a low temperature of 70 to 75°C to stabilize the reaction. The particles prepared are only small, which fact lowers the water absorption rate and water absorption capacity.

DE-A-36 28 482 relates to a process for producing highly absorptive resins by adjusting the water content of the hydrophilic polymer to 10-40% by weight and crosslinking the polymer with polyglycidyl ether having at least epoxy groups. The system used comprises ethylcellulose as a surfactant. This ethylcellulose has a low HLB value of 3-6. This prior art process only produces fine polymer particles.

In general, a highly water absorptive resin requires good water absorption rate, water absorptive capacity and gel-strength. These performances exhibit mutual opposing correlations with each other. Therefore, other performances should he sacrificed to some extent in order to improve a certain performance.

The foregoing three properties, namely the water absorptive capacity, the water absorption rate and the gel-strength in the water absorbent resin, are influenced by many factors such as polymer particle size and shape, the kinds of crosslinking agent and dispersing agent used, CMC(Critical Micelle Concentration), polymerization temperature, and so on.

In the inverse emulsion polymerization and the inverse suspension polymerization W/O systems, it is known that the use of a surfactant having a HLB value of 3∼6 make a stable system. But according to these polymerization reactions, the resultant particles are too minute and have many hydrophobic groups in their surface, and then the particulate polymer has swollen too slowly when contacted with water. Consequently, the water absorption rate and the water absorptive capacity become deteriorated. To solve the foregoing problem when a surfactant having a HLB value of 8∼12, which is known that it is much unstable in the inverse emulsion and suspension polymerization systems, is used, there is an another problem that the reaction control is difficult due to agglomeration.

On the other hand, US patent No. 4,340,706 discloses a water absorbent resin suitable for usages requiring stability in the fluid-absorbed state for a long period of time.

This resin is obtained by a process which comprises subjecting an aqueous solution of acrylic acid and an alkyl metal acrylate in an alicyclic or aliphatic hydrocarbon solvent containing a surfactant having a HLB value of 8-12 to inverse suspension polymerization. This polymerization is effected in the presence of a water soluble radical polymerization initiator to produce alkali metal acrylate polymer having an excellent salt solution absorbing ability. Consequently the obtained polymer is crosslinked with a crosslinking agent.

Systems comprising a surfactant having a HLB value of 8-12 have disadvantages. More in particular, polymer particles agglomerate and a highly heating reaction occurs in the system. In order to overcome these disadvantages, the process according to US-A-4 430 706 provides a more or less stable state by slowly adding a monomer at a relatively low temperature of 55-60°C and removing the heat of polymerization. The size of the particles obtained is however small as the system is stable, and a relatively large amount of monomers remain unreacted because of the low temperature reaction.

Representative methods for improving the water absorption rate include methods by, for example, increasing the surface area of the resin(i.e. decreasing the apparent specific gravity), crosslinking the surface of particulate polymer after polymerization, or adding inorganic materials. Among the foregoing methods, the method of adding inorganic materials such as silica has an advantage in that the dispersing rate of particulate polymer is remarkably improved in accordance with preventing agglomeration of polymer particles. However, there is a disadvantage in that it is difficult to obtain the desired water absorption rate due to poor sedimentation rate of particulate polymer. Also, in case of using the method of crosslinking the surface of particulate polymer to improve the water absorption rate, there are some disadvantages in that the minute particles formed tend to blow off and to agglomerate together, and resultingly it is difficult to obtain satisfactory water absorption rate.

### Summary of the Invention

Hereupon, the present inventors have conducted extensive studies for production of a highly water absorptive resin. As a result, it has surprisingly been found that the water absorptive resins having excellent water absorption properties can be obtained by carrying out the process according to the invention.

The process of the invention is a process for preparing a highly water absorptive resin, which comprises the following steps:
(a) suspending an aqueous solution of partially neutralized alkali metal acrylate wherein about 50 to 100% by mole of the carboxyl group has been neutralized to its alkali metal salt and at least one water soluble radical polymerization initiator, in a hydrocarbon solvent containing a surfactant having a HLB value of 8∼12 ;
(b) subjecting the mixture to inverse suspension polymerization while adjusting the internal temperature of the reactor successively to 70°C, to 60∼67°C, to 70°C, and then to 75°C, to obtain the irregular water absorptive resin by means of phase-transition ;
(c) during or after the polymerization, separating the moisture from the produced polymer by azeotropic distillation to reduce the water content to about 15 to 55% by weight, and then filtering to remove the solvent ;
(d) adding a crosslinking agent having two or more reactive epoxy groups which is dissolved in methanol, wherein the amount of the crosslinking agent is 0.005∼15% by weight based on the produced polymer, to subject surface-crosslinking for 1∼2 hours at temperatures of 70 to 85°C ; and
(e) washing with methanol, filtering, and drying at temperatures of 90 to 175°C to obtain the water absorptive resin.

The present inventors have found the surprising fact that a highly water absorptive resin can be obtained in an irregular shape by carrying out polymerization with use of a surfactant having a HLB value of 8∼12 which is generally considered to be unsuitable for inverse suspension polymerization system and inverse emulsion polymerization system, while adjusting the reaction temperature successively to 70°C, to 60∼67°C, to 70°C and to 75°C, to undergo phase-transition.

Irregular shape resin thus prepared has an increased-surface area, and then its water absorption rate and gel-strength become improved. Also, in this polymerizing system the viscosity of resin is suddenly increased by developing the Trommsdorff effect to conduct phase-transition.

### BRIEF DESCRIPTION OF THE INVENTION

In order to better understand the invention reference will be made to the accompanying drawing in which :
Figure 1 is a microphotograph (X 200) of the water absorptive resin having irregular shape according to Example 1 ; and
Figure 2 is a microphotograph (X 200) of the spherical water absorptive resin according to Comparative Example 3.

### DETAILED DESCRIPTION OF THE INVENTION

According to the present invention, there are provided highly water absorptive resins and a process for preparing them.

In the present invention, the polymer used includes, for example, polyacrylic acid, polymethacrylic acid, or a copolymer thereof with maleic acid, acrylamide, 2-acrylamido-2-methylpropylsulfonic acid, 2-methacryloylethane sulfonic acid, 2-hydroxyethyl methacylate, or the like in a certain proportion. The most preferred polymer is polyacrylic acid.

The alkali metal acrylate can be obtained by neutralizing acrylic acid with an alkali metal hydroxide such as lithium hydroxide, sodium hydroxide, potassium hydroxide and calcium hydroxide. The degree of neutralization is preferably about 50∼100% by mole, more preferably about 65∼80% by mole. When the neutralization degree is lower than 50% by mole, the hydroxide concentration would be decreased and the electronic density in the water would also be small and further, the penetration intensity would he decreased. Thus the desired highly water absorptive resin would not he obtainable. The concentration of the monomer thus produced, i.e., the alkali metal acrylate and the unneutralized acrylic acid, is about 20∼70% by weight based on the total weight of the composition. The preferred monomer concentration is about 40∼60% by weight.

As the surfactant used in this invention, any surfactant having a HLB value of 8∼12 and containing many hydrophilic groups may be used, and preferably sorbitan monolaurate (SPAN 20®, manufactured by ICI Americas Inc., HLB value = 8.6) and Ryoto Sugar Ester S-970® (manufactured by Mitsubishi-Kasei Food Corporation(MFC), HLB value = 9), particulary Ryoto Sugar Ester S-970^{R} give good results.

In general, in case that a surfactant having a HLB value of 8∼ 12, which has more hydrophilic groups than hydrophobic groups, is used in the polymerization of the water absorptive resin, the possibility to contact with the hydrophilic monomer of the starting material is increased. As a result, the inverse emulsion polymerization system and the inverse suspension polymerization system become very unstable, therefore, the viscosity is suddenly increased due to the Trommsdorff effect and the reaction is very rapidly progressed. Consequently, it causes agglomeration of particulate polymer because of the Weigenberg effect which is a characteristic in polymeric materials and also the heat of polymerization is emitted rapidly.

However, according to the present invention the disadvantageous effects as described above can be prevented and the polymerization system becomes stable. In addition, it is unnecessary to add separately a crosslinking agent during polymerizaton, and a self-crosslinking reaction and a pseudo-crosslinking reaction take place to improve greatly the absorbency of the polymer obtained. Also, the surface of the polymer thus obtained is crosslinked with the use of a crosslinking agent having three or more reactive groups such as epoxy group in the presence of an inert solvent such as methanol, and the polymer absorbs the methanol containing the crosslinking agent and is swollen somewhat. Therefore, the crosslinking agent permeates easily into the surface of particulate polymer and resultingly the surface-crosslinking reaction takes place well, whereby the water absorption rate and gel-strength of the resin becomes improved remarkably.

The water soluble radical polymerization initiator used in this invention is one well known in the art of polymer chemistry, for example, ammonium persulfate, potassium persulfate, hydrogen peroxide, and the like. The preferred water soluble radical polymerization initiator is potassium persulfate. The above-mentioned polymerization initiator may be used alone or in admixture of two or more.

The hydrocarbon solvent used in the present invention is an aliphatic or aromatic hydrocarbon, which has a boiling point of 30∼200°C. Examples thereof are n-hexane, n-heptane, cyclohexane, and the like, and the perferred solvent is cyclohexane.

The surface-crosslinking agent used is one having at least three or more reactive groups such as epoxy groups, for example, glycerol polyglycidyl ether, trimethylol propane polyglycidyl ether and sorbitol polyglycidyl ether, preferably glycerol polyglycidyl ether. In crosslinking step(d), methanol as an inert solvent is preferably used for more improving the surface-crosslinking effect.

It is possible to explain this phenomenon, as follows :
(1) one by first occuring phase-transition from a system having greater mechanical force by agitation than viscous force to another system wherein mechanical force and viscous force are balanced, and followed by phase-transition to the system having greater mechanical force by agitation than viscous force again.
(2) one by first occuring phase-transition from a system (namely W/O system) wherein main phase is hydrophobic, to a water and oil equilibrium phase system, and followed by phase-transition to the hydrophobic system (W/O system) again.

As a result of this phase-transition effect the water absorptive capacity is further improved due to chemical self-crosslinking in particles during polymerization, and physical pseudo-crosslinking by trapped entanglement of main chain. Moreover, as a result of raising the polymerization temperature successively as mentioned above the shape of polymer is not spherical but irregular.

The process for preparation of the highly water absorptive resin according to the present invention is described in detail as follows :

### (1) Preparation of the partially neutralized alkali metal acrylate

Acrylic acid is charged in a reactor equipped with a condenser, a dropping funnel and a stirrer. An alkali metal hydroxide is dissolved in water in a beaker separately to form an alkali metal hydroxide solution. The alkali metal hydroxide solution is fed dropwise to the reactor through the dropping funnel while being kept at less than 30°C and neutralizing 50∼100% by mole of the acrylic acid to produce an equivalent amount of an alkali metal acrylate having the degree of neutralization of 50∼100% by mole. When the neutralization step is conduct at a temperature of over 45°C, one of reactants could be polymerized.

### (2) Inverse suspension polymerization step

A surfactant and a hydrocarbon solvent are charged in a reactor with the condenser having a Dean-Stark equipment, a presure-adjusting dropping funnel and a stirrer, and then the temperature of the reactor is raised to 70∼80°C. A mixture of the partially neutralized alkali metal acrylate prepared in step(1) and a water soluble radical polymerization initiator is incrementally charged to the reactor. The temperature is adjusted successively to 70°C, to 60∼67°C, to 70°C, and then to 75°C to form a clot of gel and consequently irregular shapes are obtained due to phase-transition.

### (3) Azeotropic distilling step

The resultant polymer contains a large amount of water so that it is difficult for the crosslinking agent to react at the surface of the polymer. Therefore, the moisture contained in the polymer is separated therefrom by azeotropic distillation to reduce the water content of the polymer to about 15 to 55% by weight. At this time, when the water content is less than 15% weight, it is uneconomic. On the other hand, when the water content is over 55% by weight, it is decreased the effect of surface-crosslinking.

After thus separating the water from the polymer, the internal temperature of the reactor is maintained room temperature. The solvent is removed by filtration to obtain the polymer.

### (4) Surface-crosslinking step

The water content adjusted polymer produced by step(3) is crosslinked by the crosslinking agent so as to improve water absorption rate and gel-strength. The polymer having a water content of 15∼55% by weight and the solution of the crosslinking agent and methanol are charged to a reactor equipped with a condense, a stirrer, and a dropping funnel. At a temperature of 75 to 80°C, the surface-crosslinking reaction is carried out for 1∼ 2 hours. The polymer thus treated is cooled to room temperature and the methanol is removed by filtering method.

### (5) Washing and drying step

To remove residual monomers, the obtained polymer is washed twice with methanol and filtered. The filtered polymer is dried at a temperature of 90 to 175°C and passed through a 40-mesh wire gauze to obtain the water absorptive resin in particles having a uniform size. The obtained resin is provided to measure the water absorption rate and the water absorptive capacity by filtering method.

The present invention is described in detail by the following examples, but it should be noted that the invention is not limited by these examples.

The term "the water absorptive capacity used in the present invention means a value determined according to the following procedure : To 2,000g of deionized water is added 1g of the dried water absorptive resin, and water is absorbed by the polymer for 30 minutes, after which the polymer is collected by filtration with a 80-mesh metallic wire gauze, the volume of the swollen polymer obtained is measured, and the value is taken as the deionized water absorption capacity.

The saline solution-absorption rate is a value (g/g) calculated from the weight of the saline solution(0.9% by weight aqueous sodium chloride solution) which is absorbed by the polymer for, respectively, 1, 3 and 5 minutes.

### Example 1

200g of cyclohexane and 2.0g of Ryoto Sugar Ester S-970^{R} were charged in a 1ℓ four-necked round-bottomed flask equipped with a stirrer, a condenser having a Dean-Stark equipment, a pressure adjusting-dropping funnel and a nitrogen gas inlet pipe.

The nitrogen gas was introduced into the flask so as to remove oxygen in the flask, and then the flask was heated to 75°C in oil bath.

Separately, 36g of acrylic acid was charged in another 1ℓ four-necked round-bottomed flask equipped with a stirrer, a condenser and a dropping funnel.

While cooling it to room temperature, 65g of aqueous sodium hydroxide solution prepared by dissolving 15g of sodium hydroxide in 50g of distilled water was slowly fed dropwise thereto to prepare an aqueous solution of 75% neutralized sodium acrylate.

After 0.12g of potassium persulfate was dissolved thoroughly in 3.88g of distilled water in a 100mℓ beaker, it was mixed with the previously prepared sodium acrylate solution and stirred. The mixture was rapidly added to the flask containing cyclohexane and the surfactant through the pressure adjusting-dropping funnel over about 10 minutes.

After keeping the internal temperature of the flask to 70°C, the temperature was changed successively to 64°C, to 70°C again, and to 75°C (The temperature of the oil both was as follows : 85°C → 73°C → 85°C → 88°C), to undergo phase-transition. Thereafter, the temperature of the oil bath was held to 90°C for an hour to complete the polymerization, 28g of water was separated from the produced polymer by azeotropic distillation to reduce the water content to 40% by weight. After cooling the flask to room temperature, cyclohexane was filtered off, and the polymer was introduced to the flask equipped with a condenser, stirrer and dropping funnel.

0.375g of Epok 812® (glycerol polyglycidyl ether) and 200g of methanol were mixed in a 300mℓ beaker. After the mixture was added to the flask containing the polymer through a dropping funnel, it was reacted at 80°C for an hour.

Thereafter, the temperature was cooled to room temperature, and the solution was filtered to obtain the polymer.

The obtained polymer was washed with 40g of methanol, filtered, and dried to obtain a water absorptive resin having an irregular shape. And the dried water absorptive resin of irregular shapes was passed through a 20-mesh wire gauze to obtain a water absorptive resin in particles having a uniform size.

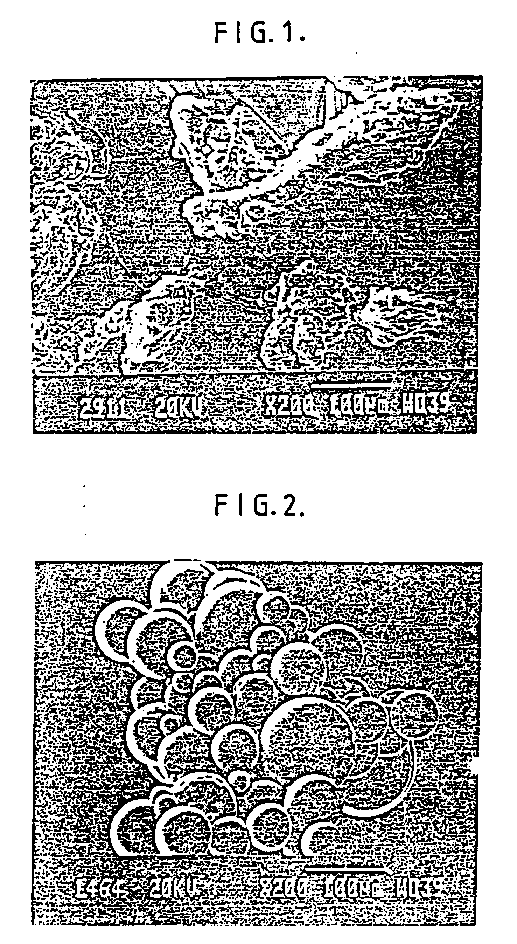

The results are shown in Table 1. The microphotograph of Example 1 is shown in Figure 1.

### Example 2

A water absorptive resin was produced in the same manner as described in Example 1 except that 0.25g of Epok 812 was used.

The results are shown in Table 1.

### Example 3

A water absorptive resin was produced in the same manner as described in Example 1 except that 0.425g of Epok 812 was used.

The results are shown in Table 1.

### Example 4

A water absorptive resin was produced in the same manner as described in Example 1 except that 0.625g of Epok 812 was used.

The results are shown in Table 1.

### Example 5

A water absorptive resin was produced in the same manner as described in Example 1 except that 36g of acrylic acid was neutralized to 70% by adding 14g of sodium hydroxide and 47.1g of distilled water in the polymerization step.

The results are shown in Table 1.

### Example 6

A water absorptive resin was produced in the same manner as described in Example 1 except that 36g of acrylic acid was neutralized to 65% by adding 13g of sodium hydroxide and 46.9g of distilled water in the polymerization step.

The results are shown in Table 1.

### Example 7

A water absorptive resin was produced in the same manner as described in Example 1 except that 36g of acrylic acid was neutralized to 60% by adding 12g of sodium hydroxide and 46.6g of distilled water in the polymerization step.

The results are shown in Table 1.

### Comparative Example 1

A comparative sample was produced in the same manner as described in Example 1 except that 28g of water was first separated from the polymer to reduced the water content to 40% by weight, followed by the drying step.

The results are shown in Table 1.

### Comparative Example 2

A comparative sample was produced in the same manner as described in Example 1 except that 28g of water was first separated from the polymer to reduced the water content to 40% by weight, and the polymer was washed twice with 40g of methanol, followed by the drying step.

The results are shown in Table 1.

### Comparative Example 3

A comparative sample was produced in the same manner as described in Example 1 except that the temperature of oil bath was maintained to 65°C during the polymerization step.

The results are shown in Table 1 and the microphotograph is shown in Figure 2.

### Comparative Example 4

A comparative sample was produced in the same manner as described in Example 7 except that 28g of water was first separated from the polymer to reduce the water content to 40% weight, followed by the drying step.

The results are shown in Table 1.

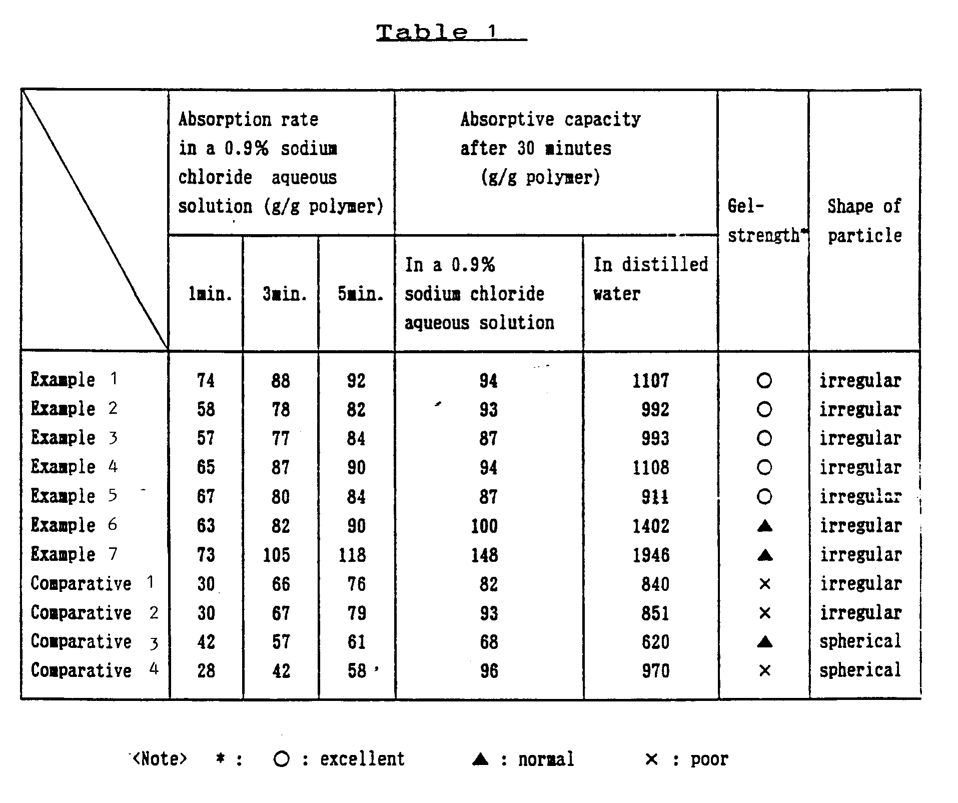

## Claims

1. A process for producing a highly water absorptive amorphous resin, which comprises
(a) suspending an aqueous solution of partially neutralized alkali metal acrylate wherein about 50 to 100% by mole of the carboxyl group has been neutralized to its alkali metal salt and at least one water soluble radical polymerization initiator, in a hydrocarbon solvent containing a surfactant having a HLB value of 8∼12;
(b) subjecting the mixture to inverse suspension polymerization while adjusting the internal temperature of the reactor successively to 70°C, to 60∼67°C, to 70°C, and then to 75°C, to obtain the irregular water absorptive resin by means of phase-transition;
(c) during or after the polymerization, separating the moisture from the produced polymer by azeotropic distillation to reduce the water content to about 15 to 55% by weight, and filtering to remove the solvent;
(d) adding a cross-linking agent having two or more reactive epoxy groups which is dissolved in methanol, wherein the amount of the cross-linking agent is 0.005∼15% by weight based on the produced polymer, to subject surface-cross-linking for 1∼2 hours at temperatures 70 to 85°C; and
(e) washing with methanol, filtering, and drying at temperatures of 90 to 175°C to obtain the irregular water absorptive resin.

2. The process according to claim 1, wherein the surfactant is sorbitan monolaurate.

3. The process according to claim 1 or 2, wherein the hydrocarbon solvent is selected from the group consisting of n-hexane, n-heptane and cyclohexane.

4. The process according to any one of the claims 1-3, wherein the hydrocarbon solvent is cyclohexane.

5. The process according to any one of the claims 1-4, wherein the water soluble radical polymerization initiator is selected from the group consisting of ammonium persulfate, potassium persulfate and hydrogen peroxide.

6. The process according to any one of the claims 1-5, wherein the water soluble radical polymerization initiator is potassium persulfate.

7. The process according to any one of the claims 1-6, wherein the cross-linking agent is selected from the group consisting of ethylene glycol diglycidyl ether, glycerol diglycidyl ether, glycerol polyglycidyl ether, trimethylol propane polyglycidyl ether and sorbitol polyglycidyl ether.

8. The process according to any one of the claims 1-7, wherein the partially neutralized alkali metal acrylate is prepared by addition of an alkali metal hydroxide such as sodium hydroxide, lithium hydroxide or potassium hydroxide.

## Patentansprüche

1. Verfahren zur Herstellung eines hoch wasserabsorbierenden amorphen Harzes, umfassend
(a) das Suspendieren einer wässrigen Lösung eines teilweise neutralisierten Alkalimetallacrylats, wobei ungefähr 50 bis 100 Mol-% der Carboxylgruppen unter Bildung ihrer Alkalimetallsalze neutralisiert worden sind, und wenigstens einen wasserlöslichen radikalischen Polymerisations-Initiator in einem Kohlenwasserstoff-Lösungsmittel, enthaltend einen oberflächenaktiven Stoff mit einem HLB-Wert von 8 ∼ 12;
(b) die Anwendung einer inversen Suspensions-Polymerisation auf die Mischung, während die Innentemperatur des Reaktors nacheinander auf 70 °C, auf 60∼67 °C, auf 70 °C und dann auf 75 °C eingestellt wird, wodurch das irreguläre wasserabsorbierende Harz durch Phasentransfer erhalten wird;
(c) während oder nach der Polymerisation das Abtrennen der Feuchtigkeit vom hergestellten Polymer durch azeotrope Destillation, wodurch der Wassergehalt auf ungefähr 15 bis 55 Gew.-% reduziert wird, und das Filtrieren, wodurch das Lösungsmittel entfernt wird;
(d) das Hinzufügen eines Vernetzungsmittels mit zwei oder mehr reaktiven Epoxygruppen, das in Methanol gelöst ist, wobei die Menge an Vernetzungsmittel 0,005 ∼ beträgt, um 1 ∼ 2 Stunden lang bei Temperaturen von 70 bis 85 °C eine Oberflächen-Vernetzung durchzuführen; und
(e) das Waschen mit Methanol, Filtrieren und Trocknen bei Temperaturen von 90 bis 175 °C, wodurch das irreguläre wasserabsorbierende Harz erhalten wird.

2. Verfahren nach Anspruch 1, wobei das oberflächenaktive Mittel Sorbitanlaurat ist.

3. Verfahren nach Anspruch 1 oder 2, wobei das Kohlenwasserstoff-Lösungsmittel aus der aus n-Hexan, n-Heptan und Cyclohexan bestehenden Gruppe ausgewählt ist.

4. Verfahren nach einem der Ansprüche 1 bis 3, wobei das Kohlenwasserstoff-Lösungsmittel Cyclohexan ist.

5. Verfahren nach einem der Ansprüche 1 bis 4, wobei der wasserlösliche radikalische Polymerisations-Initiator aus der aus Ammoniumpersulfat, Kaliumpersulfat und Wasserstoffperoxid bestehenden Gruppe ausgewählt ist.

6. Verfahren nach einem der Ansprüche 1 bis 5, wobei der wasserlösliche radikalische Polymerisations-Initiator Kaliumpersulfat ist.

7. Verfahren nach einem der Ansprüche 1 bis 6, wobei das Vernetzungsmittel aus der aus Ethylenglycoldiglycidylether, Glycerindiglycidylether, Glycerolpolyglycidylether, Trimethylolpropanpolyglycidylether und Sorbitolpolyglycidylether bestehenden Gruppe ausgewählt ist.

8. Verfahren nach einem der Ansprüche 1 bis 7, wobei das teilweise neutralisierte Alkalimetallacrylat durch Zugabe eines Alkalimetallhydroxids wie Natriumhydroxid, Lithiumhydroxid oder Kalimhydroxid hergestellt wird.

## Revendications

1. Procédé pour la production d'une résine amorphe à haute absorption d'eau, procédé qui comprend:
(a) suspension d'une solution aqueuse d'acrylate de métal(aux) alcalin(s) partiellement neutralisée et caractérisée en ce qu'environ 50 à 100% en moles de la fonction carboxylique ont été neutralisés en son sel de métal alcalin et en ce qu'il y a au moins un amorceur de polymérisation radicalaire hydrosoluble dans un solvant hydrocarbure contenant un tensioactif ayant un ELH (équilibre lipophile hydrophile) compris entre 8 et 12 ;
(b) exposition du mélange à une réaction de polymérisation en suspension inverse, alors que la température interne du reacteur est successivement ajustée à 70°C, à 60-67°C, à 70°C, puis à 75°C pour qu'une résine irrégulière absorbant l'eau soit obtenue grâce à une transition de phases ;
(c) séparation, pendant ou après la polymérisation, de l'eau du polymère produit, cette séparation étant effectuée par une distillation azéotrope afin que la teneur en eau soit réduite à environ 15-55% en poids, puis filtration pour l'élimination du solvant ;
(d) adjonction d'un agent de réticulation possédant deux ou plusieurs fonctions réactives époxy et dissous dans du méthanol, caractérisée en ce que la quantité de l'agent de réticulation est de 0,005 à 15% en poids sur la base du polymère produit, afin que la réticulation de la surface soit soumise 1 à 2 heures à une température de 70 à 85°C ; et
(e) lavage au méthanol, filtration, puis séchage à une température de 90 à 175°C pour l'obtention de la résine irrégulière absorbant l'eau.

2. Procédé selon la revendication 1, caractérisé en ce que le tensioactif est le monolaurate de sorbitan.

3. Procédé selon la revendication 1 ou 2, caractérisé en ce que le solvant hydrocarbure est choisi dans un groupe consistant en le n-hexane, le n-heptane et le cyclohexane.

4. Procédé selon l'une quelconque des revendications 1 à 3, caractérisé en ce que le solvant hydrocarbure est le cyclohexane.

5. Procédé selon l'une quelconque des revendications 1 à 4, caractérisé en ce que l'amorceur de polymérisation radicalaire hydrosoluble est choisi dans un groupe consistant en le persulfate d'ammonium, le persulfate de potassium et le peroxyde d'hydrogène.

6. Procédé selon l'une quelconque des revendications 1 à 5, caractérisé en ce que l'amorceur de polymérisation radicalaire hydrosoluble est le persulfate de potassium.

7. Procédé selon l'une quelconque des revendications 1 à 6, caractérisé en ce que l'agent de réticulation est choisi dans un groupe consistant en l'éther diglycidylique de l'éthylèneglycol, l'éther diglycidylique du glycérol, l'éther polyglycidylique du glycérol, l'éther polyglycidylique du triméthylolpropane et l'éther polyglycidylique du sorbitol.

8. Procédé selon l'une quelconque des revendications 1 à 7, caractérisé en ce que l'acrylate de métal alcalin partiellement neutralisé est préparé par l'adjonction de l'hydroxyde d'un métal alcalin comme l'hydroxyde de sodium, l'hydroxyde de lithium ou l'hydroxyde de potassium.
